(19) Europäisches Patentamt
European Patent Office
Office européen des brevets

(11) Veröffentlichungsnummer: **0 013 389**
**A2**

# (12) EUROPÄISCHE PATENTANMELDUNG

(21) Anmeldenummer: **79105253.3**

(22) Anmeldetag: **18.12.79**

(51) Int. Cl.³: **C 08 F 20/60**
C 07 D 265/32

(30) Priorität: **12.01.79 DE 2901003**

(43) Veröffentlichungstag der Anmeldung:
**23.07.80 Patentblatt 80/15**

(84) Benannte Vertragsstaaten:
**BE DE FR GB**

(71) Anmelder: **BASF Aktiengesellschaft**
**Carl-Bosch-Strasse 38**
**D-6700 Ludwigshafen(DE)**

(72) Erfinder: **Naarmann, Herbert, Dr. Dipl.-Chem.**
**Haardtblick 16**
**D-6719 Wattenheim(DE)**

(72) Erfinder: **Degen, Hans-Juergen, Dr. Dipl.-Chem.**
**Schillerstrasse 6**
**D-6143 Lorsch(DE)**

## (54) Morpholongruppenhaltige Polymerisate.

(57) Die Erfindung betrifft morpholongruppenhaltige Polymerisate, welche die Struktureinheiten der allgemeinen Formel:

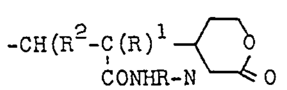
,

worin die Reste R bis $R^2$ übliche organische Reste sind, enthalten. Diese Polymerisate werden durch Homo- oder Copolymerisation der entsprechenden olefinisch ungesättigten Acrylamidverbindung erhalten. Die erfindungsgemäßen Polymerisate sind leicht anfärbbar und können zur Herstellung von Formkörpern, Überzügen, Klebstoffen und zum Veredeln von Papier und Textilien verwendet werden.

EP 0 013 389 A2

Morpholongruppenhaltige Polymerisate

Die Erfindung betrifft neue Polymerisate mit K-Werten von 20 bis 140.

Der Erfindung lag die Aufgabe zugrunde, Polymerisate zu schaffen, die Morpholongruppen in den Seitenketten enthalten.

Die Aufgabe wird gelöst durch Polymerisate mit K-Werten von 20 bis 140, die Struktureinheiten der allgemeinen Formel (I)

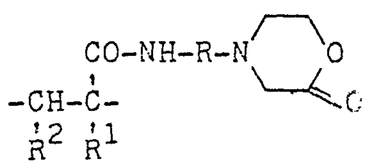

(I)

enthalten, worin

R = $C_1$- bis $C_{18}$-Alkylen, $C_6$- bis $C_{20}$-Cycloalkylen, -Arylen, -Alkylarylen oder Biphenylenäther

$R^1$ = H oder $CH_3$

$R^2$ = H, COOH oder COOR

bedeuten.

Die Polymerisate können entweder Homopolymerisate mit wiederkehrenden Einheiten der allgemeinen Formel (I) oder Copolymerisate sein, welche die Einheiten der Formel (I) und andere Struktureinheiten einpolymerisiert enthalten, die von einer oder mehreren copolymerisierbaren olefinisch ungesättigten Verbindungen abstammen.

Bevorzugt sind Polymerisate, deren Rest R ein Äthylen-, Propylen-, Butylen- oder Phenylen-Rest ist.

Fre/BL

Bevorzugt sind ferner Polymerisate, bei denen der Rest $R^2$ ein COOH- oder $COOCH_3$-Rest ist.

Bevorzugte Copolymerisate sind diejenigen, die als copolymerisierbare olefinisch ungesättigte Verbindung Acrylsäureäthylester, Acrylsäure, Acrylnitril, Butadien, Äthylen, Styrol oder Acrylsäuremethylester einpolymerisiert enthalten.

Unter Polymerisaten mit K-Werten von 20 bis 140, die Struktureinheiten der allgemeinen Formel (I)

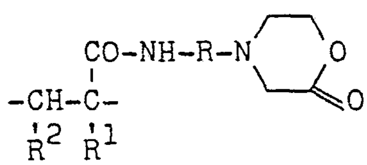

enthalten, werden oligomere oder makromolekulare Stoffe verstanden, die ganz oder im wesentlichen oder teilweise aus wiederkehrenden Einheiten der Formel

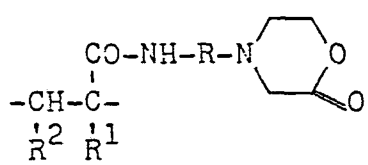

bestehen, worin der Formelteil $-[CH_2-C]-$ ein Teil der Polymerisatkette ist. Die Polymerisate enthalten in der Seitenkette als Substituenten des Morpholonrest. Die allgemeine Formel beschreibt also Hompolymerisate, die nur die oben angeführten Strukturelemente (I) enthalten, als auch Copolymerisate mit einpolymerisierten copolymerisierbaren olefinisch ungesättigten Verbindungen.

Unter K-Wert der Polymerisate wird die technische Kenngröße verstanden, die zur Charakterisierung des Polymerisationsgrads eines Polymerisats üblich ist. Die K-Werte der erfindungsgemäßen Polymerisate wurden nach H. Fikentscher, Cellulosechemie 13 (1932) Seiten 58 bis 64 und 71 bis 74, eingewichtsprozentig in Dimethylformamid bei einer Temperatur von 25°C gemessen; dabei bedeutet $K = k \cdot 10^3$.

Die erfindungsgemäßen Polymerisate werden hergestellt durch Homopolymerisation der Verbindungen (II)

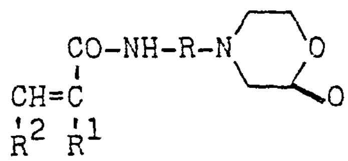

(II)

in denen R, $R^1$ und $R^2$ die oben angeführte Bedeutung haben oder durch Copolymerisation einer Verbindung der allgemeinen Formel II mit einem oder mehreren, mit II copolymerisierbaren olefinisch ungesättigten Monomeren in Gegenwart von Radikale bildenden Initiatoren.

Verbindungen der Formel II werden hergestellt beispielsweise aus den Säureanhydriden oder Säurechloriden der entsprechenden olefinisch ungesättigten Derivate durch Umsetzung

mit 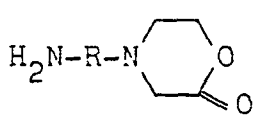 .

Dazu werden z.B. in Dioxan bei Temperaturen zwischen +10°C und 100°C im Molverhältnis 1:1 die beiden Komponenten zusammengegeben und nach einer Reaktionszeit von 1 bis 5 Stunden vom abgeschiedenen NaCl abfiltriert und die Dioxanlösung, die das erfindungsgemäße Monomer enthält, aufgearbeitet. Die Reaktion wird analog den Vorschriften entsprechend R.H. Yocum und E.B. Nyquist in "Functional Monomers" Vol. I, Seite 6 (1973) New York, durchgeführt.

Besonders geeignete olefinisch ungesättigte, morpholongruppenhaltige Monomere der Verbindungsklasse II sind die Verbindungen III bis VIII:

Molgewicht

III 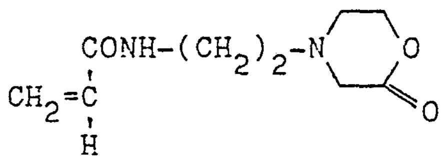 (198)

IV 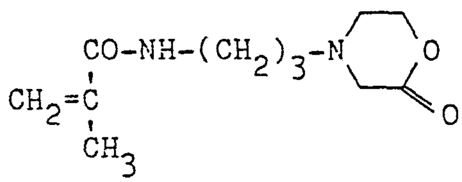 (226)

V 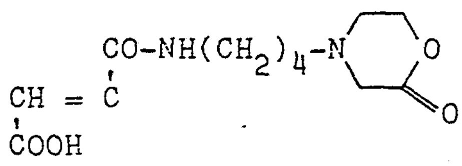 (270)

VI 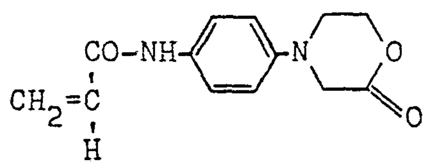 (246)

VII 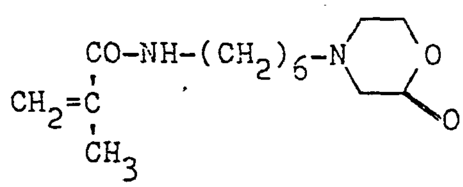 (268)

VIII 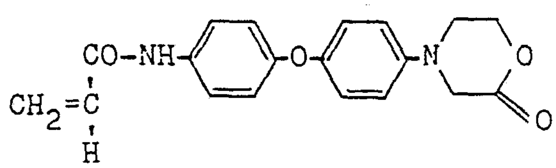

wobei die Verbindung III am besten geeignet ist.

Die Monomeren der Formel II können jeweils für sich allein polymerisiert werden. Selbstverständlich ist es auch mög-

lich, Gemische verschiedener dieser Verbindungen zu copolymerisieren. Außerdem sind sie der Copolymerisation mit anderen olefinisch ungesättigten Monomeren zugänglich.

Geeignete olefinisch ungesättigte Comonomere, die gegebenenfalls mit den genannten Morpholonderivaten der Formeln II copolymerisiert werden können, sind beispielsweise Olefine, wie Äthylen, Propylen, Butadien, Isopren; Styrol und substituierte Styrole, wie $\alpha$-Methylstyrol, p-Chlorstyrol und p-Methylstyrol; Acryl- und Methacrylsäureester, beispielsweise des Methanols, Äthanols, Butanols oder Äthylcyclohexanols, Äthylenglykols, Propylenglykols-1,2, Butenylglykols-1,3 oder des Butylenglykols-1,4; Acryl- und Methacrylsäureamid und substituierte Amide, wie N-Methylolacrylamid oder deren Äther, wie N-Methylolacrylamidbutyläther, N-Methylolmethacrylamidmethyläther; Acryl- und Methacrylnitril; Vinylester wie Vinylacetat, Vinylpropionat; Vinyläther, wie Methyl-, Äthyl,-, oder Alkylvinyläther mit Alkylresten mit 3 bis 6 Kohlenstoffatomen; ferner Fumar-, Malein- oder Itaconsäure, Ester diese Säuren und Maleinsäureanhydrid. Von den vorstehend genannten Verbindungen können auch gleichzeitig zwei oder mehr mit den genannten Morpholonderivaten (II) copolymerisiert werden.

Für die Herstellung von Copolymerisaten kann der Anteil an olefinisch ungesättigten Morpholonderivaten (II) im Monomerengemisch in weiten Grenzen schwanken, beispielsweise zwischen 1 und 99, insbesondere zwischen 5 und 80, vorzugsweise zwischen 8 und 60 Gewichtsprozent, bezogen auf das Gesamtgewicht der Monomeren.

Zur Auslösung der Polymerisation werden übliche Radikale bildende Initiatoren verwendet. Geeignete Initiatoren sind beispielsweise Wasserstoffperoxid, organische Hydroperoxide und Peroxide, wie Caproylperoxid, Lauroylperoxid, t-Butyl-

perbenzoat, Dicumylperoxid, p-Menthanhydroperoxid, Cumolhydroperoxid, Bernsteinsäureperoxid, ferner unter Polymerisationsbedingungen in Radikale zerfallende aliphatische Azoverbindungen, wie 2,2'-Azo-bis-2,4-dimethylvaleronitril, 2,2'-Azo-bis-isobutyronitril und analoge Azonitrile, die beispielsweise in J. Hine "Reaktivität und Mechanismus in der organischen Chemie", Verlag Georg Thieme, Stuttgart (1960), Seite 412, aufgeführt sind, sowie übliche Redoxkatalysatorsysteme, wie die Systeme Kalium- oder Ammoniumpersulfat und Ascorbinsäure, Natriumhydrosulfit oder Eisen-II-salze.

Geeignet sind ferner die als Radikalbildner bekannten Chelate von Übergangsmetallen, wie Chelate des Mangan(III), Kobalt(III), Kupfer(II) und Cer(IV). Im allgemeinen werden als Chelatbildner 1,3-Dicarbonylverbindungen verwendet. Als Beispiele seien genannt Mangan(III)-acetylacetonat und Kobalt(III)-acetessigester. Außerdem kann die Polymerisation auch durch Strahlung, gegebenenfalls in Gegenwart von Sensibilisatoren, wie Benzoinderivaten, ausgelöst werden.

Die Initiatoren werden im allgemeinen in einer Menge von 0,05 bis 5 Gewichtsprozent, vorzugsweise 0,1 bis 1,0 Gewichtsprozent, bezogen auf die Monomerenmenge, verwendet. Die optimale Menge und der optimal wirksame Initiator lassen sich durch Versuche leicht ermitteln. Die Polymerisation kann in Substanz durchgeführt werden. Vorteilhaft arbeitet man jedoch in Gegenwart von Lösungs- oder Verdünnungsmitteln. Geeignet sind beispielsweise Ketone, wie Methyläthyl- oder Methylpropylketon, Äther wie Diäthyläther, Tetrahydrofuran oder Dioxan; aliphatische, cycloaliphatische oder aromatische Kohlenwasserstoffe, wie Hexan, Heptan, Cyclohexan, Benzol oder Toluol, gut geeignet ist auch Dimethylformamid.

Die für eine Vielfalt anderer Monomerer oder Monomerengemische üblichen Suspension- oder Lösungspolymerisationsverfahren sind auch für das neue Verfahren geeignet. Auch bezüglich der gegebenenfalls verwendeten Hilfsmittel, wie Dispergiermittel, Schutzkolloide und dergleichen unterscheidet sich das neue Verfahren nicht von bekannten.

Die Polymerisation kann in einem weiten Temperaturbereich, etwa zwischen 0 und 150°C, vorzugsweise zwischen 50 und 120°C bei Reaktionszeiten von 1 bis 20 Stunden, vorzugsweise 2 bis 10 Stunden durchgeführt werden. Man arbeitet im allgemeinen bei Atmosphärendruck, doch können auch höhere Drucke angewandt werden. Insbesondere bei Copolymerisationen mit niedrigsiedenden Comonomeren ist die Verwendung von höheren Drucken angezeigt, um eine ausreichende Konzentration des Comonomeren im Reaktionsgemisch zu bewirken.

Die Copolymerisation der olefinisch ungesättigten Verbindungen (II) mit Äthylen oder Butadien wird vorteilhaft in aliphatischen oder aromatischen Lösungsmitteln durchgeführt, indem man die miteinander copolymerisierbaren Monomeren in das Lösungsmittel, das einen Initiator enthält, einbringt und bei erhöhtem Druck, bei Äthylen als Comonomeren bis etwa 2000 atü, polymerisiert.

Die Copolymerisation mit Acrylsäureestern wird zweckmäßig in aromatischen oder aliphatischen Kohlenwasserstoffen unter den für die Polymerisation von Acrylsäureestern bekannten Bedingungen durchgeführt.

Aufgrund der Morpholongruppe sowie gegebenenfalls der Carboxyl-, bzw. Estergruppe in der Seitenkette sind diese Polymerisate reaktiv und gegebenenfalls weiteren Reaktionen zugänglich. Die Polymerisate können beispielsweise intermolekular vernetzt werden.

Die erfindungsgemäßen Polymerisate, die K-Werte von 20 bis 140, vorzugsweise von 50 bis 90 aufweisen, sind aufgrund ihres Gehaltes an Morpholongruppen leicht anfärbbar. Sie werden beispielsweise für die Herstellung von Formkörpern, schlagfesten Massen, Überzügen oder Klebemitteln, auch im Gemisch mit anderen Kunststoffen, z.B. mit Polyäthylen, Polypropylen oder mit Mischpolymerisaten aus Vinylacetat und Äthylen, verwendet. Wegen ihrer oberflächenaktiven Eigenschaften sind die Polymerisate unter anderem auch zum Veredeln von Papier und Textilien geeignet.

Von besonderem technischen Interesse sind ferner Copolymerisate der olefinisch ungesättigten morpholongruppenhaltigen Derivate (II) mit Acrylsäureestern. Diese Produkte sind löslich, hochmolekular, mit Pigmenten mischbar. Solche Copolymerisate sind vorzüglich als Lacke verwendbar.

Die in den Beispielen genannten Teile und Prozente beziehen sich auf das Gewicht. Sofern kein anderes Lösungsmittel genannt wird, wurden die K-Werte jeweils eingewichtsprozentig in Dimethylformamid nach der Vorschrift von H. Fikentscher, Cellulosechemie 13, (1932), Seite 58 bestimmt.

Beispiel 1

Acrylsäureäthylester und die Verbindung III werden in bestimmten Mengenverhältnissen gemischt, mit jeweils 0,1 Gewichtsprozent Azo-bis-isobuttersäurenitril versetzt und 2 Stunden auf 70°C erwärmt.

Die Copolymerisate werden mit Methanol gefällt, mit Methanol gewaschen und im Vakuumtrockenschrank 10 Stunden bei 60°C und 12 mm Hg getrocknet. Die erhaltenen Ergebnisse sind in der folgenden Tabelle zusammengestellt.

| Nr. | Acrylsäure-äthylester Teile | Verbindung III Teile | Umsatz % | K-Wert % | N(%) | Anteil von Verb. III im Copolymerisat in Gew.% |
|---|---|---|---|---|---|---|
| a | 9,9 | 0,1 | 99 | 74 | 0,15 | ca. 1,0 |
| b | 9,5 | 0,5 | 98 | 79 | 0,4 | ca. 5 |
| c | 9,0 | 1,0 | 99 | 78 | 1,4 | ca. 10 |
| d | 8,5 | 1,5 | 97,5 | 76 | 2,1 | ca. 15 |
| e | 8,0 | 2,0 | 98 | 69 | 2,8 | ca. 20 |
| f | 7,0 | 3,0 | 96,5 | 76 | 4,2 | ca. 28 |
| g | 6,0 | 4,0 | 98 | 73 | 5,6 | ca. 40 |
| h | 5,0 | 5,0 | 98,5 | 79 | 7,1 | ca. 50 |
| i | 4,0 | 6,0 | 98 | 82 | 8,4 | ca. 60 |
| j | 2,5 | 7,5 | 98,5 | 71,5 | 10,4 | 74 |
| k | 1,0 | 9,0 | 97,5 | 72 | 12,5 | 87,5 |

Beispiel 2

4 Teile Acrylnitril und 6 Teile Verbindung IV werden in Gegenwart von 0,1 Teilen Azo-bis-iso-buttersäurenitril 2 Stunden bei 70°C polymerisiert. Das Copolymerisat besitzt einen K-Wert von 82 und enthält 60 Gewichtsprozent an einpolymerisierter Verbindung IV. Der Umsatz liegt bei 98,5 %.

Beispiel 3

Auf eine Lösung von 150 Teilen Toluol, 10 Teilen Verbindung V und 1 Teil Azo-bis-isobutyronitril wird unter Rühren 8 Stunden bei 90°C soviel Butadien aufgepreßt, daß der Gasraum 6 atü beträgt. Das Copolymerisat besitzt nach dieser Reaktionszeit einen Festgehalt von 28 Gewichtsprozent, einen K-Wert von 74 und enthält 12 Gewichtsprozent Verbindung V.

Beispiel 4

Es wird analog dem Beispiel 3 gearbeitet, jedoch das Butadien durch Äthylen ersetzt. Man erhält bei einer Reaktionszeit von 8 Stunden, einer Reaktionstemperatur von 90°C und einem Äthylendruck im Gasraum von 265 atü eine Lösung mit einem Festgehalt von 20 Gewichtsprozent. Der K-Wert des Copolymerisats, das einen Anteil von ca. 10 Gewichtsprozent der Verbindung V enthält, beträgt 46 (gemessen eingewichtsprozentig in Dekahydronaphthalin).

Beispiel 5

Eine Lösung aus 100 Teilen Toluol, 10 Teilen Verbindung VI, 290 Teilen Styrol und 3 Teilen Azo-bis-isobutyronitril wird unter Rühren 8 Stunden auf 90°C erwärmt. Man erhält 300 Teile eines Copolymerisates mit einem K-Wert von 78 (0,5 gewichtsprozentig in Toluol) und einem Gehalt an Verbindung VI von ca. 3,3 Gewichtsprozent.

Beispiel 6

6 Teile Verbindung VII werden in Gegenwart von 0,01 Teilen Azo-bis-isobutyronitril 2 Stunden auf 70°C erhitzt. Man erhält 5,9 Teile Homopolymerisat der Verbindung VI mit einem K-Wert von 74 (eingewichtsprozentig in Dioxan).

Beispiel 7

Wird analog den Angaben des Beispiels 6 gearbeitet, jedoch die Verbindung VI als Monomeres verwendet, so erhält man 6,0 Teile eines Homopolymerisats vom K-Wert 79.

Beispiel 8

Erfolgt die Polymerisation nach Beispiel 7, wird jedoch Verbindung VIII verwendet, so erhält man 6,0 Teile Hompolymerisat mit einem K-Wert von 73.

Beispiel 9

Wird wie im Beispiel 7 beschrieben gearbeitet, jedoch die Verbindung III als Monomeres verwendet, so erhält man 6,0 Teile eines Hompolymerisates mit einem K-Wert von 69.

Patentansprüche

1. Polymerisate mit K-Werten von 20 bis 140, die Struktureinheiten der allgemeinen Formel (I)

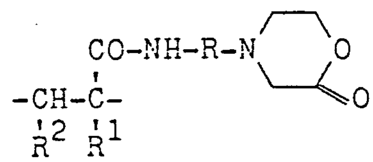

(I)

enthalten, worin

R = $C_1$- bis $C_{18}$-Alkylen, $C_6$- bis $C_{20}$-Cycloalkylen, -Arylen, -Alkylarylen oder Biphenylenäther,

$R^1$ = H oder $CH_3$

$R^2$ = H, COOH oder COOR

bedeuten.

2. Polymerisate nach Anspruch 1, dadurch gekennzeichnet, daß es Homopolymerisate mit wiederkehrenden Einheiten der allgemeinen Formel (I) sind.

3. Polymerisate nach Anspruch 1, dadurch gekennzeichnet, daß es Copolymerisate sind, welche Einheiten der Formel (I) und andere Struktureinheiten einpolymerisiert enthalten, die von einer oder mehreren copolymerisierbaren olefinisch ungesättigten Verbindungen abstammen.